(19) **Europäisches Patentamt / European Patent Office / Office européen des brevets**

(11) **EP 3 812 429 B1**

(12) **EUROPEAN PATENT SPECIFICATION**

(45) Date of publication and mention
of the grant of the patent:
**18.05.2022   Bulletin 2022/20**

(21) Application number: **19206094.5**

(22) Date of filing: **30.10.2019**

(51) International Patent Classification (IPC):
**C09C 1/00** *(2006.01)*          **A61K 8/02** *(2006.01)*
**A61K 8/26** *(2006.01)*          **A61Q 1/02** *(2006.01)*
**A61K 8/04** *(2006.01)*          **A61K 8/19** *(2006.01)*
**A61K 8/25** *(2006.01)*          **A61Q 1/00** *(2006.01)*
**A61Q 19/00** *(2006.01)*

(52) Cooperative Patent Classification (CPC):
**C09C 1/0021; A61K 8/0266; A61K 8/044;**
**A61K 8/19; A61K 8/25; A61K 8/26; A61Q 1/00;**
**A61Q 1/02; A61Q 19/00;** A61K 2800/262;
A61K 2800/412; A61K 2800/43; A61K 2800/621;
A61K 2800/63; C01P 2004/04;          (Cont.)

(54) **TRANSPARENT COLORLESS FLAKY PIGMENT, PROCESS FOR THE PRODUCTION ANDUSE THEREOF**

TRANSPARENTES FARBLOSES PLÄTTCHENFÖRMIGES PIGMENT, VERFAHREN ZUR HERSTELLUNG UND VERWENDUNG DAVON

PIGMENT EN PAILLETTES INCOLORE TRANSPARENT, PROCÉDÉ DE PRODUCTION ET UTILISATION CORRESPONDANTS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **02.11.2018   EP 18204077**

(43) Date of publication of application:
**28.04.2021   Bulletin 2021/17**

(73) Proprietor: **Merck Patent GmbH
64293 Darmstadt (DE)**

(72) Inventors:
• **Watanabe, Yukitaka
IWAKI 970-8045 (JP)**
• **Suzuki, Ryuta
IWAKI 971-8188 (JP)**

(56) References cited:
**WO-A1-2012/031664          US-A1- 2007 179 241
US-A1- 2011 064 779          US-A1- 2016 199 492**

(52) Cooperative Patent Classification (CPC): (Cont.)
C01P 2004/54; C01P 2006/10; C01P 2006/17;
C09C 2200/1004; C09C 2220/106

**Description**

The present invention is directed to a transparent colorless flaky pigment, to a process for the production thereof as well as to the use of such a pigment, in particular as a soft-focus filler in cosmetic formulations.

Flaky powders such as mica or clay materials are known to be useful as filler materials or as basis for filler materials in several applications, in particular for cosmetic formulations. Since these materials sometimes exhibit not all of the requested characteristics of filler materials by themselves, they are occasionally either mixed with other ingredients or coated therewith.

In cosmetic applications, properties such as providing a matte skin surface, soft skin-feeling, natural look and good spreadability are highly desired. In addition, light-diffusing pigments are often used which should provide a good balance of transparency, scattering and reflection to minimize the visibility of wrinkles, which becomes more and more important for make-ups, anti-wrinkle products and skin correctors, especially for elder people.

Unfortunately, it is complicated to adjust a good balance of wrinkle concealment and natural look within a single filler pigment, since wrinkle concealment is due to diffuse light scattering of the pigments which usually causes a whitish appearance of the skin as well.

Thus, transparent filler pigments providing wrinkle concealment as well as a transparent natural look would be of great advantage.

So far, filler pigments in the market provide often merely a good transparency or an excellent wrinkle concealment, but there is still a need for a filler pigment being capable to provide both properties with one single filler pigment only when applied in a cosmetic formulation.

The ability of a filler to impart a particularly attractive and natural appearance on the skin while, at the same time, exhibiting a satisfying light scattering (diffuse reflection) to conceal wrinkles and skin impurities is often referred to as the soft-focus effect.

Several products are on the market which fulfill more or less either the one or the other optical request for a cosmetic filler powder. Often, these products are based on mica or other phyllosilicates being used either as neat powders or coated or mixed with materials such as silica, titanium dioxide, alumina, barium sulfate, etc. BiOCI or BN powders or alumina particles are used as well. The powder particles may exhibit a flaky, spherical or granular shape, or a mixture of at least two thereof. US 2007/179241 A1 solves the problem of providing a care, makeup or treatment composition for keratinous material such as the skin and/or the lips which is able to provide improved soft focus effect properties upon application.

US 2016/199492 A1 is directed to improve the cosmetic and medical phototherapeutic treatment in such a way that uniform illumination of the entire skin is achieved.

US 2011/064779 A1 solves the problem to provide Improved effect pigments. A particular desire is to provide effect pigments having improved soft focus and a pleasant feeling on the skin.

Based on the disadvantages of the filler powders mentioned above it is the object of the present invention to provide a transparent flaky colorless pigment which is especially useful in cosmetic applications and provides a good transparency as well as a good soft-focus effect to the skin which is treated with the respective cosmetic formulation containing the filler. In addition, usual characteristics such as soft skin-feeling, good spreadability and good adhesion to the skin should be provided as well.

Furthermore, it is an object of the present invention to provide a process for the production of a filler pigment having the characteristics as described before.

In addition, it is a further object of the present invention to provide the use of the respective pigments, in particular as filler powders in cosmetic formulations.

The object of the present invention is achieved by a transparent colorless flaky pigment, consisting of a transparent colorless flaky core particle and of a coating on the core particle, wherein the coating consists of a first and a second separate continuous sublayer, each sublayer being composed of aluminium oxide ($Al_2O_3$) and/or aluminium oxide hydroxide (AlO(OH)), the second sublayer being located on top of the first sublayer, where the coating has a density $\rho < 3.00$ g/cm$^3$ and where the density of the first sublayer is larger than the density of the second sublayer.

In addition, the object of the present invention is achieved by a process for the production of said transparent colorless flaky pigment, comprising the following steps:

- Providing an aqueous suspension of transparent colorless flaky core particles at a temperature in the range of from 45°C to 80°C;
- Adjusting a pH value in the range of from pH 4.5 to pH 9.0;
- Adding a first amount of an aqueous solution of a water soluble aluminium salt while maintaining the pH value;
- Allowing a first aluminium hydroxide sublayer to precipitate onto the flaky core particle;
- Raising the temperature of the aqueous suspension by at least 10°C;
- Adding a second amount of an aqueous solution of a water soluble aluminium salt while controlling the pH value in the range of from pH 4.5 to pH 9.0;
- Allowing a second aluminium hydroxide sublayer to precipitate onto the first aluminium hydroxide sublayer;
- Separating, washing and drying the colorless flaky core particles coated with the first and second sublayers; and
- Calcining the thus coated colorless flaky core particles at a temperature in the range of from 300°C to 1400°C, whereby the aluminium hydroxide in the first and second sublayers is converted to aluminium oxide and/or aluminium oxide hydroxide.

Furthermore, the object of the present invention is also achieved by the use of said transparent colorless flaky pigments as filler in cosmetic formulations, paints, lacquers, inks, printing inks or plastics.

The transparent colorless flaky pigments according to the present invention are based on transparent colorless flaky core particles which are provided with a coating.

In order to avoid a whitish or dull appearance of the present pigments, the core particles of the pigments must be as transparent and colorless as possible. To this end, materials such as $Al_2O_3$ flakes, talc flakes, $SiO_2$ flakes, glass flakes, BN flakes or synthetic mica flakes are preferably used as core particles. Natural mica flakes are less useful, since they include foreign ions such as iron ions which may impart a slightly colored appearance to them which is not of advantage in the present pigments. $Al_2O_3$ flakes are particularly preferred, especially $\alpha$-$Al_2O_3$-flakes. The transparent colorless flaky core particles for the present pigments are available in the market in several qualities and size distributions, manufactured by several companies. Particular preference is given to $\alpha$-$Al_2O_3$-flakes which are described in their characteristics and production method in US 2017/0105915 A1.

Neat $Al_2O_3$ flakes may be used as filler pigments in cosmetic formulations as well. Usually, they impart a relatively low coverage to the skin, leading to a little whitish effect only, and impart a soft skin-feeling and good adhesion to the skin as well as comfortable spreadability characteristics, but the soft-focus factor thereof is low and needs to be improved. Similar defects regarding the one or other characteristic may be observed for the other core flake materials mentioned above as well, when used in a neat form without any coating thereon.

The present inventors did therefore investigate eagerly how the good skin performance of all core particle materials mentioned above could be maintained by improving their general attractivity with respect to a good balance of transparency and soft-focus effect.

The present inventors did find that a particular porous coating onto useful transparent colorless flaky core particles can impart the desired diffuse reflection, i.e. the soft-focus effect, to them, while the transparency of the core particles could be maintained to such an extent that the resulting pigments may be regarded transparent to visible light when used in cosmetic formulations.

The coating on the transparent colorless flaky core particles is colorless as well and is composed of two continuous sublayers laying on top of each other, the first sublayer laying directly on the surface of the transparent colorless flaky core particles and, preferably, surrounding the core particles, whereas the second sublayer being located on the first sublayer and surrounding the latter.

With respect to the material composition, the two continuous sublayers are composed of the same components, namely of aluminium oxide and/or aluminium oxide hydroxide, where the content of aluminium oxide hydroxide is dependent on the production details, especially on the calcination temperature, as will be explained hereunder.

Nevertheless, the two sublayers exhibit different reflection properties, due to the different porosity in each sublayer. Based on a unique production process, two sublayers of the same material composition, but exhibiting different reflection behavior, may be achieved. Since the pore volume in each sublayer may hardly be measured, the inventors rely instead on the density of the coating comprising the two sublayers.

The theoretical density of pure $Al_2O_3$ is given as $\rho = 3.95$ or $3.94$ g/cm$^3$ in the literature, whereas the theoretical density of AlO(OH) is given as about $3.1$ g/cm$^3$. Since the coating contains pores having the density of air of $1.00$ g/cm$^3$, the density of the coating of the present pigments is smaller than the theoretical density of AlO(OH) or of $Al_2O_3$ and is $\rho < 3.00$ g/cm$^3$, in particular $\leq 2.70$ g/cm$^3$, especially $\leq 2.60$ g/cm$^3$.

The density of the coating may be calculated by determining the density of the whole pigment by a usual pycnometer, determining the weight content of the core particles and of the coating (the weight content of the core particles corresponds to the weight of the core particles used as starting materials, whereas the weight content of the coating is determined via the amount of the aluminium compound solution used for the coating procedure), determining the density of the neat core particles and calculating the density of the coating by the formula:

$$\rho_{coating} = (\rho_{pigment} - (\rho_{core\ particle} \times weight\ content\ core\ particle)) / weight\ content\ coating$$

All densities mentioned in the present disclosure rely on values determined at 20°C.

In general, the pigment according to the present invention consists of 30-70% by weight of the colorless flaky core particle and of 30-70% by weight of the coating, where the sum of the core particle and the coating is 100% by weight. Preference is given to a content of 40-60% by weight of the colorless transparent core particle and 40-60% by weight of the coating. Especially, the weight percentage of the coating is larger than that of the core. In particular, the weight content of the coating is 55 to 60% by weight, where the sum of the core particle and the coating is 100% by weight.

The size of the pore diameters and the relation of the total pore area in respect to the total cross-section area covered by each sublayer may be determined by evaluating cross-sectional TEM samples of pigments according to the present invention. The TEM samples are analyzed using a Hitachi HD-2700 STEM (scanning transmission electron microscope). The cross-sectional TEM samples are prepared using a focused ion beam (FIB). The magnification is 80.000x.

The TEM samples reveal a two-layer structure of the coating of the present pigments, consisting of a first and second sublayer.

In the overall coating, the diameter of the pores ranges from 1 to 100 nm, preferably from 5 to 80 nm, and in particular from 10 to 60 nm.

Regarding the content of the area covered by pores (pore area) in relation to the whole area of the respective sublayer, the percentage of the pore area in the observation field is examined in different observation fields and an average value is calculated based on the values for each observation field.

According to the procedure explained above, the pore area ratio relative to the total area covered by the first sublayer is 0 to 50%, preferably 0 to 10%.

In contrast, the pore area relative to the total area covered by the second sublayer is 5 to 80%, preferably 5 to 30%.

The relative pore area covered by the pores in the second sublayer is always larger than the relative pore area covered by the pores in the first sublayer. Therefore, since both sublayers are composed of the same materials, the density of the second sublayer is smaller than the density of the first sublayer.

According to a preferred embodiment of the present invention, the transparent colorless flaky core particle of the pigments is an $Al_2O_3$ flake, in particular an $\alpha$-$Al_2O_3$ flake. Most preferred are $\alpha$-$Al_2O_3$ flakes which are produced according to the process disclosed in US 2017/0105915 A1. When these alumina flakes are used, the overall density of the resulting transparent colorless flaky pigment is lowered to a value of p < 3.30 g/cm$^3$, in particular to p < 3.15 g/cm$^3$, even if the pigment consisting of the core particle and the two sublayers of the coating is composed of pure aluminium oxide, which exhibits a theoretical density of $\rho$ = 3.94 g/cm$^3$.

The porous surface of the pigments according to the invention diminishes the whiteness of the filler pigment and its regular reflection under the influence of light, but strengthens the diffuse reflection which is almost uniform in all directions. Therefore, the soft-focus effect may be ameliorated to a great extent when compared with, e.g., the action of pure alumina flakes, while the transparency of the pigments may be maintained or even ameliorated too.

According to the present invention, the particle size of the transparent colorless flaky pigments is in the range of from 2 to 30 $\mu$m, which corresponds to the $d_{90}$ value (90% by volume of pigments in a respective pigment powder exhibit a particle size lower than the value given). The particle size refers to the largest longitudinal extension of the respective pigment. Preferably, the particle size is in the range of from 5 to 25 $\mu$m. The aspect ratio (ratio of average diameter to average thickness of the pigment) is in the range of from 1.5 to 60.

The particle size as well as the particle size distribution of the pigments is often determined by a laser diffraction method. To this end, several useful apparatuses are available in the market. The particle size and particle size distribution of the present pigments is determined by an instrument of the type Malvern Mastersizer 3000 (Malvern Panalytical (UK)) according to the laser diffraction method in a standard procedure.

The soft-focus effect which is referred to herein may not only be described, but is also measurable by a certain method and may be expressed in a graph. To this end, the present inventors refer to the disclosure of US 9,250,179 B2, where the corresponding measurement method is described in detail.

Therein, it is described that a so-called soft-focus factor (SSF) may be measured by measuring the luminance value L* at an angle of 65° (= Lw) and the luminance L* in the specular angle (=Lg) (the basis for all measurements is the CIE L*a*b* color system, the measurements are done by a usual goniophotometer). The quotient Lw/Lg describes the uniformity of the scattered light distribution over the various viewing angles and is, therefore, a good predictor of the soft-focus effect of the respective pigment. In an ideal case, the factor Lw/Lg is 1 for the best soft-focus effect available.

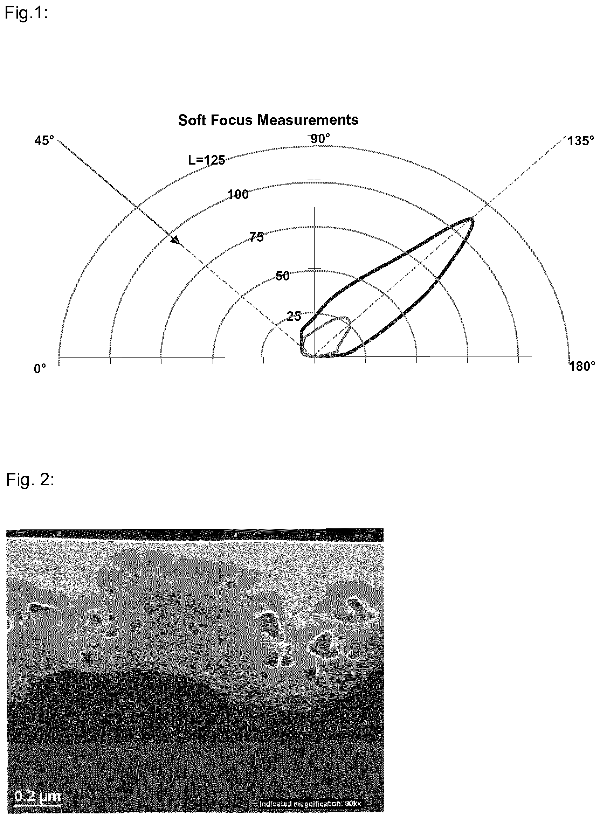

According to this method, the soft-focus factor achieved by the present pigments is about 0.5, whereas the soft-focus factor of a pure alumina flake is only about 0.15 (see graph of Fig. 1).

The present invention is also directed to a process for the production of the transparent colorless flaky pigments as described above, the process comprising the following steps:

- Providing an aqueous suspension of transparent colorless flaky core particles at a temperature in the range of from 45°C to 80°C;
- Adjusting a pH value in the range of from pH 4.5 to pH 9.0;
- Adding a first amount of an aqueous solution of a water soluble aluminium salt while maintaining the pH value;
- Allowing a first aluminium hydroxide sublayer to precipitate onto the flaky core particle;

- Raising the temperature of the aqueous suspension by at least 10°C;
- Adding a second amount of an aqueous solution of a water soluble aluminium salt while controlling the pH value in the range of from pH 4.5 to pH 9.0;
- Allowing a second aluminium hydroxide sublayer to precipitate onto the first aluminium hydroxide sublayer;
- Separating, washing and drying the colorless flaky core particles coated with the first and second sublayers; and
- Calcining the thus coated colorless flaky core particles at a temperature in the range of from 300°C to 1400°C, whereby the aluminium hydroxide in the first and second sublayers is converted to aluminium oxide and/or aluminium oxide hydroxide.

Preferably, the pH value in the first and second precipitation step is adjusted in the range of from pH 6.0 to pH 8.0.

The temperature range for the whole coating process is between 45°C and 105°C, while the temperature difference between the coating temperature for precipitation of the first sublayer and the coating temperature for precipitation of the second sublayer is at least 10 degrees and at most 40 degrees, in particular it is between 15 and 35 degrees centigrade. This means that between the first and second precipitation step for forming the first and second sublayer the temperature must be raised by at least 10 to 40 degrees, in particular by 15 to 35 degrees centigrade, in order to assure that the porosity of the second sublayer will be higher than that of the first sublayer in the resulting product. Accordingly, the density of the second sublayer is smaller than the density of the first sublayer in the resulting transparent colorless flaky pigment of the present invention.

The temperature range for calcination determines the percentual content of aluminium oxide hydroxide and aluminium oxide in the resulting product. In general, the calcination temperature is in the range of from 300°C to 1400°C in order to assure that the aluminium hydroxide ($Al(OH)_3$) precipitated in the first and second sublayer is converted at least to aluminium oxide hydroxide ($AlO(OH)$), the conversion starting at 300°C. The higher the calcination temperature, the higher the content of aluminium oxide will be. Therefore, the preferred calcination temperature is in the range of from 500°C to 1200°C.

As water soluble aluminium salts, those selected from the group consisting of aluminium sulfate, aluminium chloride, aluminium nitrate, sodium aluminate, and mixtures of at least two of them are preferably used.

The pH value may be adjusted by adding sodium hydroxide, potassium hydroxide, sodium carbonate, potassium carbonate or ammonia in an appropriate amount as usual and appropriate.

The transparent colorless flaky core particles which are preferably used in the present process are $Al_2O_3$ flakes, in particular the $\alpha$-$Al_2O_3$ flakes as described above, although other flaky base materials such as talc flakes, $SiO_2$ flakes, glass flakes, BN flakes or synthetic mica flakes may be used as well.

The present invention is also directed to the use of the pigments described above, namely to the use of the respective pigments as filler in cosmetic formulations, paints, lacquers, inks, printing inks or plastics. Although the present pigments are especially useful in cosmetic applications according to their pronounced transparency and soft-focus effect, they may also be used as fillers in technical applications such as described above, where the particular transparency thereof is of advantage, combined with a high oil absorbency which is due to the porosity of the coating.

In cosmetic formulations, the pigments according to the present invention may be used in an amount of from 0.5 to 95% by weight, based on the weight of the formulation, preferably of from 1 to 80% by weight and in particular of from 5 to 70% by weight. It is generally dependent on the specific application. The pigments according to the present invention may be employed in decorative as well as in care cosmetics. They are particularly suitable for products and formulations in skin care products and anti-wrinkle products such as make-ups, compact powders, lose powders, etc.

Preferably,

- emulsions contain 0.1-30 % by weight, in particular 1-15 % by weight,
- pigment-containing emulsions comprise 0.1-50% by weight, in particular 1-15 % by weight, depending on the texture,
- toothpastes contain 0.1-60 % by weight, in particular 1-50 % by weight,
- water-free oil/wax-based products comprise 0.1-75 % by weight, in particular 0.5-65 % by weight,
- powder products contain 0.1-95 % by weight, in particular 1-75 % by weight,

of the pigments according to the invention, based on the formulation as a whole.

The pigments according to the present invention can be mixed with commercially available state-of-the-art fillers which are neither restricted in their material composition nor in their shape. Fillers which may be mentioned are, for example, natural and synthetic mica, glass beads or glass powder, nylon powder, polymethylmethacrylate powders, pure or filled melamine resins, talc, glasses, kaolin, oxides or hydroxides of aluminium, magnesium, calcium or zinc, BiOCl, barium sulfate, calcium sulfate, calcium carbonate, magnesium carbonate, carbon, boron nitride and physical or chemical combinations of these substances.

It goes without saying that the cosmetic formulation containing the present pigments may comprise further usual ingredients (besides optional color imparting organic or inorganic pigments) which may comprise at least one absorbent, astringent, antimicrobial substance, antioxidant, antiperspirant, antifoaming agent, antidandruff active ingredient, antistatic, binder, biological additive, bleaching agent, chelating agent, deodorant, emollient, emulsifier, emulsion stabilizer, dye, humectant, film former, filler, odor imparting substance, flavorant, insect repellent, preservative, anticorrosion agent, cosmetic oil, solvent, oxidant, vegetable constituent, buffer substance, reducing agent, surfactant, propellent gas, opacifier, UV filter and UV absorber, denaturing agent, viscosity regulator, perfume or vitamin.

Examples for such usually used cosmetic raw materials and auxiliaries are, inter alia, oils, fats, waxes, film formers, surfactants, antioxidants, such as, for example, vitamin C or vitamin E, stabilisers, odour intensifiers, silicone oils, emulsifiers, solvents, such as, for example, ethanol, or ethyl acetate or butyl acetate, preservatives and auxiliaries which generally determine applicational properties, such as, for example, thickeners and rheological additives, such as, for example, bentonites, hectorites, silicon dioxides, Ca silicates, gelatines, high-molecular-weight carbohydrates and/or surface-active auxiliaries, etc.

The cosmetic formulations comprising the pigments according to the invention can belong to the lipophilic, hydrophilic or hydrophobic type. In the case of heterogeneous formulations having discrete aqueous and nonaqueous phases, the pigments according to the invention may in each case be present in only one of the two phases or alternatively distributed over both phases.

The pH of the formulations can be between 1 and 14, preferably between 2 and 11 and particularly preferably between 5 and 8.

The pigments according to the invention may furthermore also be combined with cosmetic active ingredients. Suitable active ingredients are, for example, insect repellents, inorganic UV filters, such as, for example, $TiO_2$, UV A/BC protective filters (for example OMC, B3 and MBC), also in encapsulated form, anti-ageing active ingredients, vitamins and derivatives thereof (for example vitamin A, C, E, etc.), self-tanning agents (for example DHA, erythrulose, inter alia), and further cosmetic active ingredients, such as, for example, bisabolol, LPO, VTA, ectoine, emblica, allantoin, bioflavonoids and derivatives thereof.

Organic UV filters are generally incorporated into cosmetic formulations in an amount of 0.5 to 10 % by weight, preferably 1 to 8 %, and inorganic filters in an amount of 0.1 to 30 %.

The cosmetic formulations comprising the pigments according to the invention may in addition comprise further conventional skin-protecting or skin-care active ingredients. These may in principle be any active ingredients known to the person skilled in the art.

Particularly preferred active ingredients are pyrimidine carboxylic acids and/or aryl oximes.

Of the cosmetic applications, particular mention should be made of the use of ectoine and ectoine derivatives for the care of aged, dry or irritated skin. Thus, European patent application EP-A-0 671 161 describes, in particular, that ectoine and hydroxyectoine are employed in cosmetic preparations, such as powders, soaps, surfactant-containing cleansing products, lipsticks, rouge, make-up, care creams and sunscreen compositions.

Amount and kind of any further ingredient is determined by the skilled person corresponding to the particular application requirement for the actual cosmetic formulation. Application forms of cosmetic formulations which may be mentioned are, for example, solutions, suspensions, emulsions, PIT (phase inversion temperature) emulsions, pastes, ointments, gels, creams, lotions, powders, soaps, surfactant containing cleansing compositions, oils, aerosols and sprays. Examples of other applications forms are sticks, shampoos and shower preparations. Any desired customary excipients, auxiliaries and, if desired, further active ingredients may be added to the cosmetic formulation.

Ointments, pastes, creams and gels may comprise the customary excipients, for example animal and vegetable fats, waxes, paraffins, starch, tragacanth, cellulose derivatives, polyethylene glycols, silicones, bentonites, silica, talc and zinc oxide, or mixtures of these substances. Powders and sprays may comprise the customary excipients, for example lactose, talc, silica, aluminium hydroxide, calcium silicate and polyamide powder, or mixtures of these substances. Sprays may additionally comprise the customary propellants, for example chlorofluorocarbons, propane/butane or dimethyl ether.

Solutions and emulsions may comprise the customary excipients, such as solvents, solubilisers and emulsifiers, for example water, ethanol, isopropanol, ethyl carbonate, ethyl acetate, benzyl alcohol, benzyl benzoate, propylene glycol, 1,3-butyl glycol, oils, in particular cottonseed oil, peanut oil, wheatgerm oil, olive oil, castor oil and sesame oil, glycerol fatty acid esters, polyethylene glycols and fatty acid esters of sorbitan, or mixtures of these substances.

Suspensions may comprise the customary excipients, such as liquid diluents, for example water, ethanol or propylene glycol, suspending agents, for example ethoxylated isostearyl alcohols, polyoxyethylene sorbitol esters and polyoxyethylene sorbitan esters, microcrystalline cellulose, aluminium metahydroxide, bentonite, agar-agar and tragacanth, or mixtures of these substances.

Soaps may comprise the customary excipients, such as alkali metal salts of fatty acids, salts of fatty acid monoesters, fatty acid protein hydrolysates, isothionates, lanolin, fatty alcohol, vegetable oils, plant extracts, glycerol, sugars, or mixtures of these substances.

Surfactant-containing cleansing products may comprise the customary excipients, such as salts of fatty alcohol sulfates, fatty alcohol ether sulfates, sulfosuccinic acid monoesters, fatty acid protein hydrolysates, isothionates, imidazolinium derivatives, methyl taurates, sarcosinates, fatty acid amide ether sulfates, alkylamidobetaines, fatty alcohols, fatty acid glycerides, fatty acid diethanolamides, vegetable and synthetic oils, lanolin derivatives, ethoxylated glycerol fatty acid esters, or mixtures of these substances.

Face and body oils may comprise the customary excipients, such as synthetic oils, such as, for example, fatty acid esters, fatty alcohols, silicone oils, natural oils, such as vegetable oils and oily plant extracts, paraffin oils, lanolin oils, or mixtures of these substances.

The cosmetic preparations may exist in various forms. Thus, they can be, for example, a solution, a water-free preparation, an emulsion or microemulsion of the water-in-oil (W/O) or oil-in-water (O/W) type, a multiple emulsion, for example of the water-in-oil-in-water (W/O/W) type, a gel, a solid stick, an ointment or an aerosol. It is also advantageous to administer ectoines in encapsulated form, for example in collagen matrices and other conventional encapsulation materials, for example as cellulose encapsulations, in gelatine, wax matrices or liposomally encapsulated. In particular, wax matrices, as described in DE-A 43 08 282, have proven favorable. Preference is given to emulsions. O/W emulsions are particularly preferred. Emulsions, W/O emulsions and O/W emulsions are obtainable in a conventional manner.

Further embodiments are oily lotions based on natural or synthetic oils and waxes, lanolin, fatty acid esters, in particular triglycerides of fatty acids, or oily-alcoholic lotions based on a lower alcohol, such as ethanol, or a glycerol, such as propylene glycol, and/or a polyol, such as glycerol, and oils, waxes and fatty acid esters, such as triglycerides of fatty acids.

Solid sticks consist of natural or synthetic waxes and oils, fatty alcohols, fatty acids, fatty acid esters, lanolin and other fatty substances.

If a preparation is formulated as an aerosol, the customary propellants, such as alkanes, fluoroalkanes and chlorofluoroalkanes, are generally used.

Cosmetic formulations having light-protection properties may comprise adjuvants, such as surfactants, thickeners, polymers, softeners, preservatives, foam stabilisers, electrolytes, organic solvents, silicone derivatives, oils, waxes, antigrease agents, dyes and/or pigments which color the composition itself or the hair, or other ingredients usually used in the cosmetic field.

In a particularly preferred embodiment, the cosmetic formulation according to the present invention comprises a transparent colorless flaky pigment which consists of a core particle of $Al_2O_3$ flakes, having a coating consisting of a first and second sublayer of aluminium oxide and/or aluminium oxide hydroxide thereon, where the coating exhibits a density and a gradient of the density in the two sublayers as described before.

In the case of the use of the present pigments in paints and coatings, all areas of application known to the person skilled in the art are possible, such as, for example, powder coatings, printing inks for gravure, offset, screen or flexographic printing, toners and for coatings in outdoor applications. The paints and coatings here can be, for example, radiation-curing, physically drying or chemically curing. A multiplicity of binders is suitable for the preparation of printing inks or liquid surface coatings, for example based on acreages, methacrylates, polyesters, polyurethanes, nitrocellulose, ethylcellulose, polyamide, polyvinyl butyrate, phenolic resins, maleic resins, starch or polyvinyl alcohol, amino resins, alkyd resins, epoxy resins, polytetrafluoroethylene, polyvinylidene fluorides, polyvinyl chloride or mixtures thereof, in particular water-soluble grades. The surface coatings can be powder coatings or water- or solvent-based coatings, where the choice of the coating constituents is part of the general knowledge of the person skilled in the art. Common polymeric binders for powder coatings are, for example, polyesters, epoxides, polyurethanes, acrylates or mixtures thereof.

In addition, the pigments according to the invention can be used in films and plastics, gift foils, plastic containers and moldings for all applications known to the person skilled in the art. Suitable plastics for the incorporation of the pigments according to the invention are all common plastics, for example thermosets or thermoplastics. The description of the possible applications and the plastics which can be employed, processing methods and additives are given, for example, in RD 472005 or in R. Glausch, M. Kieser, R. Maisch, G. Pfaff, J. Weitzel, Perlglanzpigmente [Pearlescent Pigments], Curt R. Vincentz Verlag, 1996, 83 ff., the disclosure content of which is also incorporated herein.

The pigments according to the invention are likewise suitable in the above-mentioned areas of application for use in blends with organic dyes and/or pigments, such as, for example, transparent and opaque white, coloured and black pigments, and with flake-form iron oxides, BiOCl, organic pigments, holographic pigments, LCPs (liquid crystal polymers) and conventional transparent, coloured and black luster pigments based on metal oxide-coated flakes based on mica, glass, $Al_2O_3$, $Fe_2O_3$, $SiO_2$, metal flakes, etc. The pigments according to the invention can be mixed in any ratio with commercially available pigments and fillers.

The pigments according to the invention are furthermore suitable for the preparation of flowable pigment compositions and dry preparations comprising one or more particles according to the invention, binders and optionally one or more additives. Dry preparations are also taken to mean preparations which comprise from 0 to 8 % by weight, preferably from 2 to 8 % by weight, in particular from 3 to 6 % by weight, of water and/or a solvent or solvent mixture. The dry preparations are preferably in the form of pellets, granules, chips, sausages or briquettes and have particle sizes of 0.2-80 mm. The dry preparations are used, in particular, in the preparation of printing inks and in cosmetic formulations.

Figures:

Figure 1: shows graphs pertaining to the soft-focus factor of the pigments according to example 1 and comparative example 1, determined according to the method disclosed in US 9,250,179 B2

Figure 2: shows a TEM cross-section image (x 80.000) of the pigment according to example 1, the location of the first sublayer is marked as fully covered area

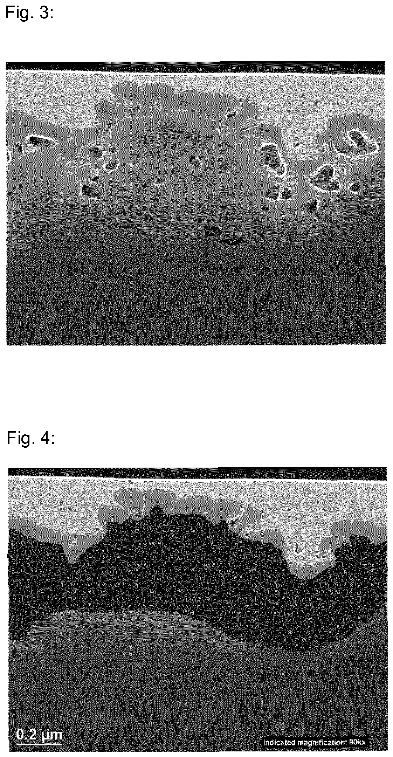

Figure 3: shows the TEM cross-section image of Fig. 2, the marked area being removed

Figure 4: shows a TEM cross-section image (x 80.000) of the pigment according to example 1, the location of the second sublayer is marked as fully covered area

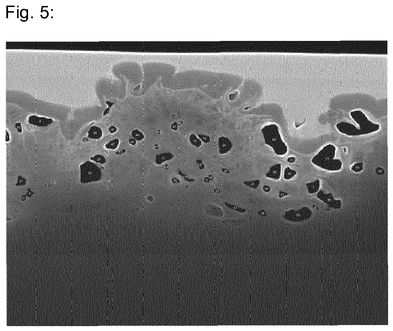

Figure 5: shows the TEM cross-section image of Fig. 4, the marked area being removed

The present invention shall be illustrated by the following examples without being restricted thereto.

Example 1:

100g alumina flakes having a mean particle size of 10 $\mu$m are suspended in 1750 ml of deionized water at a temperature of 60°C while stirring. 928 g of an aqueous aluminium sulfate solution (corresponding to 8% $Al_2O_3$) is added to the suspension at a pH of 7, the pH being maintained by the addition of NaOH. A first sublayer of aluminium hydroxide is allowed to precipitate onto the surface of the alumina flakes. The suspension is then heated up to 90°C. A further amount of 928 g of an aqueous aluminium sulfate solution (corresponding to 8% $Al_2O_3$) is then added to the suspension at a pH of 7, the pH being maintained by the addition of NaOH. A second sublayer of aluminium hydroxide is allowed to precipitate onto the surface of the first sublayer on the alumina flakes.
The solids are then filtered from the suspension, washed with deionized water and dried at a temperature of 110 °C.
The dried product is calcined at a temperature of 700°C for 10 minutes.
The resulting product exhibits a weight content of 60% of a porous coating and 40% by weight of the core alumina flake.
The porosity of the coating is examined as follows:
TEM samples of the cross section of the pigment according to example 1 are prepared (using Hitachi HD-2700 scanning transmission electron microscope and a focused ion beam). The TEM samples are evaluated at a magnification of 80.000X. Four different observation fields are evaluated for each sublayer. The overall cross-section area of the respective sublayer is determined as well as the overall area of the visible pores in the respective observation field. The results are as shown in tables 1 and 2:

Table 1:

First sublayer (Fiqs. 2 and 3):

| Observation field | total cross-section of sublayer ($nm^2$) | cross-section area of pores ($nm^2$) | ratio of pore area % |
|---|---|---|---|
| Field 1 | 363,494 | 0 | 0 |
| Field 2 | 401,446 | 6,765 | 1.69 |
| Field 3 | 342,708 | 3,914 | 1.15 |
| Field 4 | 435,797 | 1,648 | 0.38 |

The average ratio of the pore area is about 0.81% in the first sublayer.

Table 2:

second sublayer (Fiqs. 4 and 5):

| Observation field | total cross-section of sublayer ($nm^2$) | cross-section area of pores ($nm^2$) | ratio of pore area % |
|---|---|---|---|
| Field 1 | 650,042 | 90,104 | 13.86 |
| Field 2 | 644,358 | 95,272 | 14.79 |

(continued)

second sublayer (Fiqs. 4 and 5):

| Observation field | total cross-section of sublayer ($nm^2$) | cross-section area of pores ($nm^2$) | ratio of pore area % |
|---|---|---|---|
| Field 3 | 485,735 | 52,875 | 10.89 |
| Field 4 | 531,449 | 82,191 | 15.47 |

The average ratio of the pore area is about 13.75% in the second sublayer.

The pigments have an overall density of $\rho$ = 3.13 $g/cm^3$, determined by a pycnometer consisting of a specific gravity bottle of the Wadon type, product of Sibata Scientific Technology, Ltd. They exhibit a very low hiding power and a soft-focus factor SFF of 0.5, determined according to the method as described in US 9,250,179 B2 as quotient of Lw/Lg. The calculated density of the porous coating is $\rho$ = 2.59 $g/cm^3$.

Comparative example 1:

Trade product, pure white powder of uncoated $Al_2O_3$ flakes, particle size $d_{80}$ = 16 $\mu$m, density $\rho$ = 3.77 $g/cm^3$, low hiding power, SFF 0,15

A graph for comparison of the SSF of products according to example 1 and comparative example 1, respectively, is shown in Fig. 1. Whereas the small covered area pertains to the soft-focus factor of the pigments according to example 1, the larger, extended area pertains to the soft-focus factor of the pigments according to comparative example 1.

Comparative example 2:

Porous particles without core particle (2-step-method):
1750 ml of deionized water is heated to a temperature of 60°C while stirring. 928 g of an aqueous aluminium sulfate solution (corresponding to 8% $Al_2O_3$) is added at a pH of 7, the pH being maintained by the addition of NaOH. The solution is then heated up to 90°C. A further amount of 928 g of an aqueous aluminium sulfate solution (corresponding to 8% $Al_2O_3$) is added at a pH of 7, the pH being maintained by the addition of NaOH. The solids are then filtered from the resulting suspension, washed with deionized water and dried at a temperature of 110 °C.

The dried product is calcined at 700°C for 10 minutes.

Porous alumina particles having a particle size of about 8.5 $\mu$m are achieved. They exhibit a density $\rho$ =3.07 $g/cm^3$.

Comparative example 3:

Alumina flakes with porous 1-step-coating:
100g alumina flakes having a mean particle size of 10 $\mu$m are suspended in 1750 ml of deionized water at a temperature of 75°C while stirring. 1875 g of an aqueous aluminium sulfate solution (corresponding to 8% $Al_2O_3$) is added to the suspension at a pH of 7, the pH being maintained by the addition of NaOH. A layer of aluminium hydroxide is allowed to precipitate onto the surface of the alumina flakes.

The solids are then filtered from the suspension, washed with deionized water and dried at a temperature of 110 °C.

The dried product is calcined at a temperature of 1000°C for 3 hours.

The resulting product exhibits a weight content of 60% of a porous coating and 40% by weight of the core alumina flake. The overall density of the pigment is $\rho$ = 3.45 $g/cm^3$.

Comparative example 4:

Porous particles without core particle (1-step-method):
1750 ml of deionized water is heated to a temperature of 75°C while stirring. 1875 g of an aqueous aluminium sulfate solution (corresponding to 8% $Al_2O_3$) is added at a pH of 7, the pH being maintained by the addition of NaOH. The solids are then filtered from the resulting suspension, washed with deionized water and dried at a temperature of 110 °C.

The dried product is calcined at 700°c for 10 minutes.

Porous alumina particles having a particle size of about 10 $\mu$m are achieved. They exhibit a density $\rho$ =3.17 $g/cm^3$.

Evaluation of optical performance and skin-feeling effects:

The optical performance and the skin-feeling effects of pigment powders according to example 1 and comparative examples 1 to 4 are evaluated by a sensory evaluation using the following procedure:
The filler powder is applied with one finger onto the back of the hand. The same amount is used for every sample as exact as possible to allow a fair comparison. For the evaluation results, the following scales are used:

Optical effects:

| Transparency | | Soft-focus effect | |
|---|---|---|---|
| 1: | high coverage (whitish appearance) | 1: | strong luster |
| 2: | medium to high coverage | 2: | slight soft-focus |
| 3: | medium coverage | 3: | medium soft-focus |
| 4: | slight coverage | 4: | good soft-focus |
| 5: | transparent | 5: | superior soft-focus |

Skin feeling:

| Softness/Roughness | | Skin adhesion | | Slipperiness (Spreadability) | |
|---|---|---|---|---|---|
| 1: | very rough | 1: | no skin adhesion | 1: | weak |
| 2: | rough | 2: | slight | 2: | slight |
| 3: | medium | 3: | medium | 3: | medium |
| 4: | soft | 4: | medium to strong | 4: | smooth |
| 5: | very soft | 5: | strong | 5: | very smooth |

The results of the evaluation are shown in tables 3 and 4:

Table 3:

Optical effects:

| Example: | Transparency | Soft-focus effect |
|---|---|---|
| 1 | 4 | 5 |
| Comp. 1 | 3-4 | 2 |
| Comp. 2 | 4 | 5 |
| Comp. 3 | 5 | 5 |
| Comp.4 | 4 | 5 |

Regarding the optical effects, the results of example 1 and comparative examples 2 to 4 are comparable.

Table 4:

Skin-feeling:

| Example | Softness/ Roughness | Skin adhesion | Slipperiness (Spreadability) |
|---|---|---|---|
| 1 | 3 | 4 | 3 |
| Comp. 1 | 4 | 3 | 4 |
| Comp. 2 | 2 | 3 | 3 |
| Comp. 3 | 1 | 5 | 2 |
| Comp. 4 | 2 | 3 | 3 |

Regarding skin-feeling effects, the pigment powders according to comparative examples 2 to 4 are on average much worse than those of example 1 and comparative example 1. Although the trade product of comparative example 1 exhibits comparable results in skin-feeling effects, the soft-focus effect thereof is much worse than the soft-focus effect of example 1. It turns out that the pigment powders according to example 1 exhibit the best results with respect to a good balance of transparency, soft-focus effect and skin-feeling characteristics.

Use example:

Face Powder

| Ingredients | | INCI (EU) | wt. % |
|---|---|---|---|
| | | | |
| Phase A | | | |
| RonaFlair® Boroneige® SF-15 | (1) | BORON NITRIDE | 61.32 |
| RonaFlair® Mica M | (1) | MICA | 10.00 |
| Filler pigment according to example 1 | (1) | ALUMINA | 10.00 |
| Maisita Natural 9083 | (2) | ZEA MAYS | 1.60 |
| Unipure Yellow LC 182 | (3) | CI 77492 (IRON OXIDES) | 0.75 |
| Unipure Red LC 381 | (3) | CI 77491 (IRON OXIDES) | 0.19 |
| Unipure Black LC 989 | (3) | CI 77499 (IRON OXIDES) | 0.06 |
| Parteck® LUB MST | (1) | MAGNESIUM STEARATE | 1.08 |
| RonaFlair® Extender W | (1) | MICA, CI 77891 | 5.00 |
| Titan(IV)-oxid | (1) | TITANIUM OXIDE | 5.00 |
| | | | |
| | | | |
| Phase B | | | |
| Ceraphyl 368 | (4) | ETHYLHEXYL PALMITATE | 4.50 |
| Euxyl® PE 9010 | (5) | PHENOXYETHANOL, ETHYLHEXYL GLYCERIN | 0.50 |

Procedure:

Grind the titanium oxide and the iron oxides of phase A. After that, mix with the remaining ingredients of phase A until the blend is homogeneous. Then add the previously mixed phase B and mix again until the whole phase A/B is homogeneous. Fill the bulk into pans and press with the desired pressure. The pressure for pans with 59 mm diameter is approx. 210-220 bar.

The obtained face powder is a light and transparent formula. RonaFlair® Boroneige® SF-15 and RonaFlair® Mica M improve the pay-off and cause a pleasant skin-feeling while the filler pigment according to Example 1 is responsible for a good mattifying and soft-focus effect.

Suppliers:

(1) Merck KGaA, Darmstadt
(2) SLI Chemicals GmbH
(3) S. Goldmann GmbH Co. KG
(4) Ashland
(5) Schülke & Mayr GmbH

**Claims**

1. Transparent colorless flaky pigment, consisting of a transparent colorless flaky core particle and of a coating on the core particle, wherein the coating consists of a first and a second separate continuous sublayer, each sublayer being composed of aluminium oxide and/or aluminium oxide hydroxide, the second sublayer being located on top of the first sublayer, where the coating has a density $\rho < 3.00$ g/cm$^3$ and where the density of the first sublayer is larger than the density of the second sublayer.

2. Transparent colorless flaky pigment according to claim 1, wherein the colorless flaky core particle is selected from the group consisting of $Al_2O_3$ flakes, talc flakes, $SiO_2$ flakes, glass flakes, BN flakes and synthetic mica flakes.

3. Transparent colorless flaky pigment according to claim 1 or 2, wherein the colorless flaky core particle is an $Al_2O_3$ flake.

4. Transparent colorless flaky pigment according to claim 3, which has a density $\rho \leq 3.30$ g/cm$^3$.

5. Transparent colorless flaky pigment according to one or more of claims 1 to 4, wherein the pigment has a particle size in the range of from 2 to 30 $\mu$m.

6. Transparent colorless flaky pigment according to one or more of claims 1 to 5, wherein the pigment consists of 30-70% by weight of the colorless flaky core particle and 30-70% by weight of the coating, where the sum of the core particle and coating is 100% by weight.

7. Process for the production of a transparent colorless flaky pigment according to one or more of claims 1 to 6, comprising the following steps:

   - Providing an aqueous suspension of transparent colorless flaky core particles at a temperature in the range of from 45°C to 80°C;
   - Adjusting a pH value in the range of from pH 4.5 to pH 9.0;
   - Adding a first amount of an aqueous solution of a water soluble aluminium salt while maintaining the pH value;
   - Allowing a first aluminium hydroxide sublayer to precipitate onto the flaky core particle;
   - Raising the temperature of the aqueous suspension by at least 10°C;
   - Adding a second amount of an aqueous solution of a water soluble aluminium salt while controlling the pH value in the range of from pH 4.5 to pH 9.0;
   - Allowing a second aluminium hydroxide sublayer to precipitate onto the first aluminium hydroxide sublayer;
   - Separating, washing and drying the colorless flaky core particles coated with the first and second sublayers; and
   - Calcining the thus coated colorless flaky core particles at a temperature in the range of from 300°C to 1400°C, whereby the aluminium hydroxide in the first and second sublayers is converted to aluminium oxide and/or aluminium oxide hydroxide.

8. Process according to claim 7, wherein the pH value is adjusted in the range of from pH 6.0 to pH 8.0.

9. Process according to claim 7 or 8, wherein the calcining temperature is in the range of from 500°C to 1200°C.

10. Process according to one or more of claims 7 to 9, wherein the water soluble aluminium salt is selected from the group consisting of aluminium sulfate, aluminium chloride, aluminium nitrate, sodium aluminate, and mixtures of at least two of them.

11. Process according to one or more of claims 7 to 10, wherein the transparent colorless flaky core particles are $Al_2O_3$ flakes.

12. Use of a transparent colorless flaky pigment according to one or more of claims 1 to 6 as filler in cosmetic formulations, paints, lacquers, inks, printing inks or plastics.

13. Cosmetic formulation, containing a pigment according to one or more of claims 1 to 6 in an amount of from 0.5 to 95 % by weight, based on the weight of the cosmetic formulation.

14. Cosmetic formulation according to claim 13, further comprising at least one absorbent, astringent, antimicrobial substance, antioxidant, antiperspirant, antifoaming agent, antidandruff active ingredient, antistatic, binder, biological additive, bleaching agent, chelating agent, deodorant, emollient, emulsifier, emulsion stabilizer, dye, humectant, film former, filler, odor imparting substance, flavorant, insect repellent, preservative, anticorrosion agent, cosmetic oil, solvent, oxidant, vegetable constituent, buffer substance, reducing agent, surfactant, propellent gas, opacifier, UV filter and UV absorber, denaturing agent, viscosity regulator, perfume or vitamin.

15. Cosmetic formulation according to claim 13 or 14, wherein the transparent colorless flaky pigment consists of a core particle of $Al_2O_3$ flakes, having a coating of a first and second sublayer of aluminium oxide and/or aluminium oxide hydroxide thereon.

**Patentansprüche**

1. Transparentes farbloses plättchenförmiges Pigment, bestehend aus einem transparenten farblosen plättchenförmigen Kernteilchen und aus einer Beschichtung auf dem Kernteilchen, bei dem die Beschichtung aus einer ersten und einer zweiten getrennten durchgehenden Teilschicht besteht, wobei die Teilschichten jeweils aus Aluminiumoxid und/oder Aluminiumoxid-hydroxid bestehen, wobei sich die zweite Teilschicht auf der ersten Teilschicht befindet, wobei die Beschichtung eine Dichte $\rho < 3{,}00$ g/cm$^3$ aufweist und wobei die Dichte der ersten Teilschicht größer ist als die Dichte der zweiten Teilschicht.

2. Transparentes farbloses plättchenförmiges Pigment nach Anspruch 1, bei dem das farblose plättchenförmige Kernteilchen aus der Gruppe bestehend aus $Al_2O_3$-, Talkum-, $SiO_2$-, Glas-, BN- und synthetischen Glimmerplättchen ausgewählt ist.

3. Transparentes farbloses plättchenförmiges Pigment nach Anspruch 1 oder 2, bei dem das farblose plättchenförmige Kernteilchen ein $Al_2O_3$-Plättchen ist.

4. Transparentes farbloses plättchenförmiges Pigment nach Anspruch 3, das eine Dichte $\rho \leq 3{,}30$ g/cm$^3$ aufweist.

5. Transparentes farbloses plättchenförmiges Pigment nach einem oder mehreren der Ansprüche 1 bis 4, bei dem das Pigment eine Teilchengröße im Bereich von 2 bis 30 $\mu$m aufweist.

6. Transparentes farbloses plättchenförmiges Pigment nach einem oder mehreren der Ansprüche 1 bis 5, bei dem das Pigment aus 30-70 Gew.-% des farblosen plättchenförmigen Kernteilchens und 30-70 Gew.-% der Beschichtung besteht, wobei die Summe von Kernteilchen und Beschichtung 100 Gew.-% beträgt.

7. Verfahren zur Herstellung eines transparenten farblosen plättchenförmigen Pigments nach einem oder mehreren der Ansprüche 1 bis 6, umfassend die folgenden Schritte:

   - Bereitstellen einer wässrigen Suspension von transparenten farblosen plättchenförmigen Kernteilchen bei einer Temperatur im Bereich von 45°C bis 80°C;
   - Einstellen eines pH-Wertes im Bereich von pH 4,5 bis pH 9,0;
   - Zugeben einer ersten Menge einer wässrigen Lösung eines wasserlöslichen Aluminiumsalzes unter Aufrechterhaltung des pH-Wertes;
   - Ausfällenlassen einer ersten Aluminiumhydroxid-Teilschicht auf das plättchenförmige Kernteilchen;
   - Erhöhen der Temperatur der wässrigen Suspension um mindestens 10°C;
   - Zugeben einer zweiten Menge einer wässrigen Lösung eines wasserlöslichen Aluminiumsalzes unter Aussteuern des pH-Wertes im Bereich von pH 4,5 bis pH 9,0;
   - Ausfällenlassen einer zweiten Aluminiumhydroxid-Teilschicht auf die erste Aluminiumhydroxid-Teilschicht;
   - Abtrennen, Waschen und Trocknen der mit den ersten und zweiten Teilschichten beschichteten farblosen plättchenförmigen Kernteilchen und
   - Kalzinieren der derart beschichteten farblosen plättchenförmigen Kernteilchen bei einer Temperatur im Bereich von 300°C bis 1400°C, wodurch das Aluminiumhydroxid in den ersten und zweiten Teilschichten in Aluminiumoxid und/oder Aluminiumoxid-hydroxid umgewandelt wird.

8. Verfahren nach Anspruch 7, bei dem der pH-Wert im Bereich von pH 6,0 bis pH 8,0 eingestellt wird.

9. Verfahren nach Anspruch 7 oder 8, bei dem die Kalzinierungstemperatur im Bereich von 500°C bis 1200°C liegt.

10. Verfahren nach einem oder mehreren der Ansprüche 7 bis 9, bei dem das wasserlösliche Aluminiumsalz aus der Gruppe bestehend aus Aluminiumsulfat, Aluminiumchlorid, Aluminiumnitrat, Natriumaluminat und Gemischen von mindestens zwei von ihnen ausgewählt wird.

11. Verfahren nach einem oder mehreren der Ansprüche 7 bis 10, bei dem die transparenten farblosen plättchenförmigen Kernteilchen $Al_2O_3$-Plättchen sind.

12. Verwendung eines transparenten farblosen plättchenförmigen Pigments nach einem oder mehreren der Ansprüche 1 bis 6 als Füllstoff in kosmetischen Formulierungen, Farben, Lacken, Tinten, Druckfarben oder Kunststoffen.

13. Kosmetische Formulierung enthaltend ein Pigment nach einem oder mehreren der Ansprüche 1 bis 6 in einer Menge von 0,5 bis 95 Gew.-%, bezogen auf das Gewicht der kosmetischen Formulierung.

14. Kosmetische Formulierung nach Anspruch 13, ferner enthaltend mindestens ein(en) Absorptionsmittel, Adstringens, antimikrobiellen Stoff, Antioxidans, Antiperspirans, Antischaummittel, Antischuppenwirkstoff, Antistatikum, Bindemittel, biologischen Zusatzstoff, Bleichmittel, Chelatbildner, Desodorierungsmittel, Emolliens, Emulgator, Emulsionsstabilisator, Farbstoff, Feuchthaltemittel, Filmbildner, Füllstoff, Geruchsstoff, Geschmacksstoff, Insektenrepellent, Konservierungsmittel, Korrosionsschutzmittel, kosmetisches Öl, Lösungsmittel, Oxidationsmittel, pflanzlichen Bestandteil, Puffersubstanz, Reduktionsmittel, Tensid, Treibgas, Trübungsmittel, UV-Filter und UV-Absorber, Vergällungsmittel, Viskositätsregler, Parfüm oder Vitamin.

15. Kosmetische Formulierung nach Anspruch 13 oder 14, bei der das transparente farblose plättchenförmige Pigment aus einem Kernteilchen aus $Al_2O_3$-Plättchen mit darauf einer Beschichtung aus einer ersten und zweiten Teilschicht aus Aluminiumoxid und/oder Aluminiumoxid-hydroxid besteht.

## Revendications

1. Pigment pailleté incolore transparent, constitué d'une particule de cœur pailletée incolore transparente et d'un revêtement sur la particule de cœur, dans lequel le revêtement est constitué d'une première et d'une deuxième sous-couche continue séparée, chaque sous-couche étant composée d'oxyde d'aluminium et/ou d'oxyde/hydroxyde d'aluminium, la deuxième sous-couche étant située sur le dessus de la première sous-couche, où le revêtement présente une densité p < 3,00 g/cm$^3$ et où la densité de la première sous-couche est supérieure à la densité de la deuxième sous-couche.

2. Pigment pailleté incolore transparent selon la revendication 1, dans lequel la particule de cœur pailletée incolore est choisie dans le groupe constitué par les paillettes d'$Al_2O_3$, les paillettes de talc, les paillettes de $SiO_2$, les paillettes de verre, les paillettes de BN et les paillettes de mica synthétique.

3. Pigment pailleté incolore transparent selon la revendication 1 ou 2, dans lequel la particule de cœur pailletée incolore est une paillette d'$Al_2O_3$.

4. Pigment pailleté incolore transparent selon la revendication 3, qui présente une densité p ≤ 3,30 g/cm$^3$.

5. Pigment pailleté incolore transparent selon l'une ou plusieurs parmi les revendications 1 à 4, le pigment présentant une taille de particules dans la plage allant de 2 à 30 μm.

6. Pigment pailleté incolore transparent selon l'une ou plusieurs parmi les revendications 1 à 5, le pigment étant constitué de 30-70% en poids de la particule de cœur pailletée incolore et de 30-70% en poids du revêtement, la somme de la particule de cœur et du revêtement étant de 100% en poids.

7. Procédé de production d'un pigment pailleté incolore transparent selon l'une ou plusieurs parmi les revendications 1 à 6, comprenant les étapes suivantes :

   - la mise à disposition d'une suspension aqueuse de particules de cœur pailletées incolores transparentes à une température dans la plage allant de 45°C à 80°C ;
   - l'ajustement d'une valeur de pH dans la plage allant de pH 4,5 à pH 9,0;
   - l'addition d'une première quantité d'une solution aqueuse d'un sel d'aluminium hydrosoluble tout en maintenant la valeur de pH ;

- le fait de permettre à une première sous-couche d'hydroxyde d'aluminium de précipiter sur la particule de cœur pailletée ;
- l'augmentation de la température de la suspension aqueuse d'au moins 10°C ;
- l'addition d'une deuxième quantité d'une solution aqueuse d'un sel d'aluminium hydrosoluble tout en contrôlant la valeur de pH dans la plage allant de pH 4,5 à pH 9,0 ;
- le fait de permettre à une deuxième sous-couche d'hydroxyde d'aluminium de précipiter sur la première sous-couche d'hydroxyde d'aluminium ;
- la séparation, le lavage et le séchage des particules de cœur pailletées incolores revêtues avec la première et la deuxième sous-couche ; et
- la calcination des particules de cœur pailletées incolores ainsi revêtues à une température dans la plage allant de 300°C à 1400°C, l'hydroxyde d'aluminium dans la première et la deuxième sous-couche étant ainsi converti en oxyde d'aluminium et/ou en oxyde/hydroxyde d'aluminium.

8. Procédé selon la revendication 7, dans lequel la valeur de pH est ajustée dans la plage allant de pH 6,0 à pH 8,0.

9. Procédé selon la revendication 7 ou 8, dans lequel la température de calcination est dans la plage allant de 500°C à 1200°C.

10. Procédé selon l'une ou plusieurs parmi les revendications 7 à 9, dans lequel le sel d'aluminium hydrosoluble est choisi dans le groupe constitué par le sulfate d'aluminium, le chlorure d'aluminium, le nitrate d'aluminium, l'aluminate de sodium, et des mélanges d'au moins deux parmi ceux-ci.

11. Procédé selon l'une ou plusieurs parmi les revendications 7 à 10, dans lequel les particules de cœur pailletées incolores transparentes sont des paillettes d'$Al_2O_3$.

12. Utilisation d'un pigment pailleté incolore transparent selon l'une ou plusieurs parmi les revendications 1 à 6, comme charge dans les formulations cosmétiques, les peintures, les laques, les encres, les encres d'impression ou les plastiques.

13. Formulation cosmétique, contenant un pigment selon l'une ou plusieurs parmi les revendications 1 à 6 selon une quantité allant de 0,5 à 95 % en poids, sur la base du poids de la formulation cosmétique.

14. Formulation cosmétique selon la revendication 13, comprenant en outre au moins un absorbant, un astringent, une substance antimicrobienne, un antioxydant, un antiperspirant, un agent antimousse, un ingrédient actif antipelliculaire, un antistatique, un liant, un additif biologique, un agent de blanchiment, un agent chélateur, un désodorisant, un émollient, un émulsifiant, un stabilisateur d'émulsion, un colorant, un humectant, un agent filmogène, une charge, une substance conférant une odeur, un arôme, un insectifuge, un conservateur, un agent anticorrosion, une huile cosmétique, un solvant, un oxydant, un constituant végétal, une substance tampon, un agent réducteur, un agent tensioactif, un gaz propulseur, un opacifiant, un filtre UV et un absorbeur UV, un agent dénaturant, un régulateur de viscosité, un parfum ou une vitamine.

15. Formulation cosmétique selon la revendication 13 ou 14, dans laquelle le pigment pailleté incolore transparent est constitué d'une particule de cœur à base de paillettes d'$Al_2O_3$, présentant sur celle-ci un revêtement d'une première et d'une deuxième sous-couche d'oxyde d'aluminium et/ou d'oxyde/hydroxyde d'aluminium.

Fig.1:

**Soft Focus Measurements**

Fig. 2:

0.2 µm

Indicated magnification: 80kx

Fig. 3:

Fig. 4:

Fig. 5:

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007179241 A1
- US 2016199492 A1
- US 2011064779 A1
- US 20170105915 A1

- US 9250179 B2
- EP 0671161 A
- DE 4308282 A

**Non-patent literature cited in the description**

- **R. GLAUSCH ; M. KIESER ; R. MAISCH ; G. PFAFF ; J. WEITZEL.** Perlglanzpigmente [Pearlescent Pigments. Curt R. Vincentz Verlag, 1996, 83